(19) Europäisches Patentamt / European Patent Office / Office européen des brevets

(11) **EP 4 094 177 B1**

(12) **EUROPEAN PATENT SPECIFICATION**

(45) Date of publication and mention
of the grant of the patent:
**02.04.2025  Bulletin 2025/14**

(21) Application number: **20713469.3**

(22) Date of filing: **25.02.2020**

(51) International Patent Classification (IPC):
**G06F 30/27** (2020.01)     **G06N 3/08** (2023.01)
**G06F 9/455** (2018.01)     **G01N 33/2045** (2019.01)
**G06N 3/04** (2023.01)       **G06F 119/04** (2020.01)

(52) Cooperative Patent Classification (CPC):
**G06F 30/27; G06F 9/455; G06N 3/047; G06N 3/08;**
G01N 33/2045; G06F 2119/04; G06N 3/045

(86) International application number:
**PCT/US2020/019691**

(87) International publication number:
**WO 2021/173121 (02.09.2021 Gazette 2021/35)**

(54) **SYSTEM AND METHOD FOR FATIGUE RESPONSE PREDICTION**

SYSTEM UND VERFAHREN ZUR VORHERSAGE VON ERMÜDUNGSREAKTIONEN

SYSTÈME ET PROCÉDÉ POUR LA PRÉDICTION DE RÉPONSE À LA FATIGUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**30.11.2022  Bulletin 2022/48**

(73) Proprietor: **Siemens Industry Software NV
3001 Leuven (BE)**

(72) Inventors:
• **MIRABELLA, Lucia**
**Plainsboro, New Jersey 08536 (US)**
• **ARVANITIS, Elena**
**Somerville, New Jersey 08876 (US)**
• **LIU, Dehao**
**Marietta, Georgia 30067 (US)**
• **LAMMENS, Nicolas**
**3020 Herent (BE)**
• **ERDELYI, Hunor**
**3050 Oud-Heverlee (BE)**
• **LUDWIG, Christoph Ernst**
**81673 München (DE)**

(74) Representative: **Horn Kleimann Waitzhofer
Schmid-Dreyer
Patent- und Rechtsanwälte PartG mbB
Theresienhöhe 12
80339 München (DE)**

(56) References cited:
**US-A1- 2018 113 967**

• **BELFIORE ET AL: "A hybrid approach to the
development of a multilayer neural network for
wear and fatigue prediction in metal forming",
TRIBOLOGY INTERNATIONAL, ELSEVIER LTD,
AMSTERDAM, NL, vol. 40, no. 10-12, 28 August
2007 (2007-08-28), pages 1705 - 1717,
XP022218100, ISSN: 0301-679X, DOI: 10.1016/
J.TRIBOINT.2007.01.008**

**Description**

BACKGROUND

1.Field

The present disclosure relates, in general, to the field of material fatigue response prediction. Embodiments of the present disclosure are applicable, for example, for predicting fatigue life of additively manufactured parts from surface roughness parameters.

2. Description of the Related Art

Industrial use of additive manufacturing (AM) is becoming ever so common for manufacturing complex parts with high repeatability and performance. Use of AM technologies save time and resources, which are one of the advantages of AM over conventional manufacturing techniques. However, parts fabricated by AM generally have poor surface roughness. Moreover, surface roughness varies from region to region within a component based on geometry (e.g. overhang angle).

Surface quality of a part may have a significant impact on the fatigue life of the part. Surface preparation and machining techniques that reduce surface roughness have been successfully employed to extend fatigue lives of parts that are subjected to cyclic stresses. While such methods may be applicable generally to external surfaces of additively manufactured parts, the internal "as-built" surfaces of complex geometry parts may prove more challenging to modify.

Techniques have been developed for assessing the influence of surface roughness on fatigue performance. However, state of the art techniques require expensive and time-consuming experimentation and/or heavy computational resources.

The publication from Belfiore et al entitled "A hybrid approach to the development of a multilayer neural network for wear and fatigue prediction in metal forming", TRIBOLOGY INTERNATIONAL, ELSEVIER LTD, AMSTERDAM, NL, vol. 40, no. 10-12, 28 August 2007 (2007-08-28), pages 1705-1717, ISSN: 0301-679X, DOI: 10.1016/J.TRIBOINT.2007.01.008 describes a method for surface damage prediction for the case of metal forming, more precisely flow forming where a workpiece coming from a primary process is plastically deformed under the action of a roller, which influences the surface fatigue of the roller.

Patent document US 2018/113967 A1 (AGRAWAL ANKIT [US] ET AL) 26 April 2018 (2018-04-26) pertains to the prediction of fatigue strength of steel alloys comprising deriving data like steel fatigue from material databases.

SUMMARY

Briefly, aspects of the present disclosure are directed to techniques for prediction of fatigue response based on surface roughness that address at least some of the technical challenges mentioned above.

According to an aspect of the present disclosure, a computer-implemented method is provided for predicting a fatigue response of a material. The method comprises receiving a user input specifying one or more surface roughness parameters that characterize a surface of a material for which fatigue life is to be predicted. The method further comprises generating at least one realistic virtual surface profile from the specified one or more surface roughness parameters. The method further comprises predicting fatigue life of the material in dependence of a stress field applied to the generated virtual surface profile.

In accordance with specific non-limiting embodiments disclosed herein, the virtual surface profile may be generated utilizing machine learning based generative models, frequency pattern matching, Autoregressive Moving Average (ARMA) models, or combinations thereof. Furthermore, in accordance with specific non-limiting embodiments disclosed herein, the prediction of the fatigue life may be carried out utilizing finite element analysis based simulations, machine learning methods, or combinations thereof.

Other aspects of the present disclosure implement features of the above-described method in computing systems and computer program products.

BRIEF DESCRIPTION OF THE DRAWINGS

The foregoing and other aspects of the present disclosure are best understood from the following detailed description when read in connection with the accompanying drawings. To easily identify the discussion of any element or act, the most significant digit or digits in a reference number refer to the figure number in which the element or act is first introduced.

FIG. 1 is a block diagram of an example of a computing system wherein aspects of the present disclosure may be implemented

FIG. 2 is a flowchart broadly illustrating a method for predicting fatigue response based on surface roughness.

FIG. 3 illustrates a 1D surface curve showing microscopic asperities.

FIG. 4 is a flowchart illustrating an example method for generating a virtual surface profile by a machine learning based generative model.

FIG. 5 is a flowchart illustrating an example method for generating a virtual surface profile by an Autoregressive Moving Average (ARMA) model.

FIG. 6 is a flowchart illustrating an example method for generating a virtual surface profile based on frequency pattern matching.

FIG. 7 is a flowchart illustrating a method for predicting fatigue response based on surface roughness according to a first example implementation.

FIG. 8 is a flowchart illustrating a method for predicting fatigue response based on surface roughness according to a second example implementation.

FIG. 9 is a flowchart illustrating a method for predicting fatigue life based on surface roughness according to a third example implementation.

DETAILED DESCRIPTION

Aspects of the present disclosure relate to prediction of fatigue response based on surface roughness. Surface roughness is generally poor for parts fabricated by additive manufacturing (AM), which may significantly affect the fatigue performance of such parts. To assess surface roughness, one can perform surface height measurements, which may be expensive to perform and/or not accessible to all users. To assess the fatigue life of a part, a known approach is to not only quantify the surface roughness, but also generate a huge database of fatigue data corresponding to the different surface conditions. Generating such database currently is done through experiments, meaning that a large number of test specimens need to be manufactured with different surface conditions and then tested. Such an experimental campaign may be time consuming and expensive. Moreover, one needs to repeat it for different AM machines, process settings and powders. An alternative to the experimental approach is to predict the effect of surface roughness on fatigue life through simulation techniques. However, such techniques may be computationally intensive and time consuming. Furthermore, such techniques also require surface measurements, which may be difficult and expensive to perform.

The techniques disclosed herein address at least the above described problems by automatically generating a realistic virtual surface profile based on user-specified surface roughness parameters and predicting the corresponding fatigue property in dependence of a stress field applied to the virtual surface profile. The proposed techniques rely on finite element analyses (FEA), advanced machine learning (ML) methods, or combinations thereof. Using the proposed techniques, one can obtain in an efficient numerical way the fatigue property corresponding to any user defined surface roughness parameters, thereby avoiding expensive and time-consuming experimental methods. Aspects of present disclosure may be embodied in a computer-aided engineering (CAE) or computer-aided manufacturing (CAM) package.

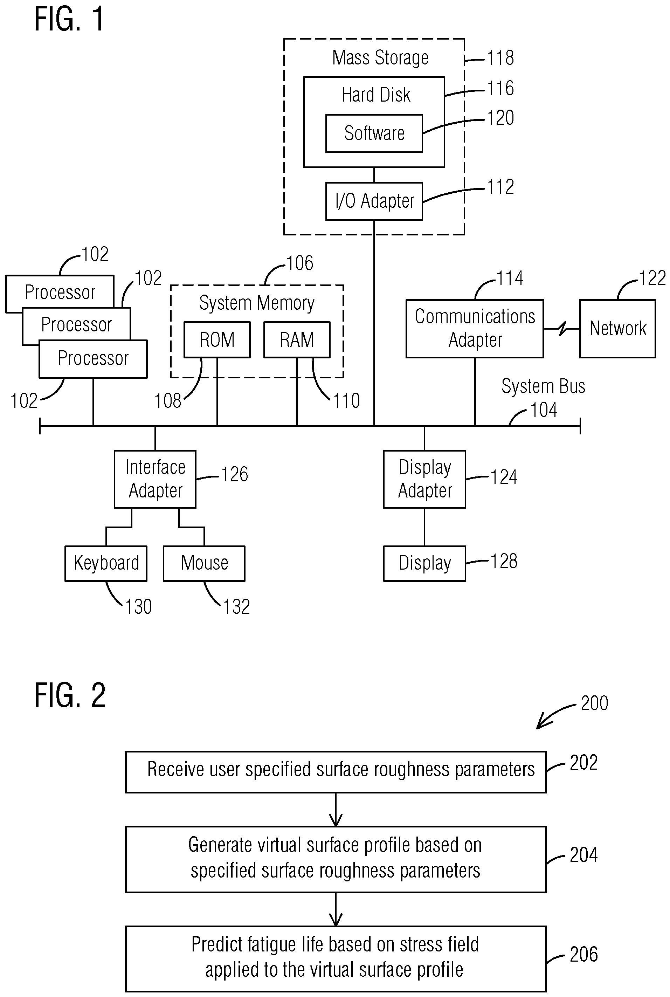

Turning now to FIG. 1, a computing system 100 is generally shown wherein aspects of the present disclosure may be implemented. The computing system 100 can be an electronic, computer framework comprising and/or employing any number and combination of computing devices and networks utilizing various communication technologies. The computing system 100 may be easily scalable, extensible, and modular, with the ability to change to different services or reconfigure some features independently of others. The computing system 100 may be, for example, a server, desktop computer, laptop computer, tablet computer, or smartphone. In some examples, computing system 100 may be a cloud computing node.

Computing system 100 may be described in the general context of computer executable instructions, such as program modules, being executed by a computing system. Generally, program modules may include routines, programs, objects, components, logic, data structures, and so on that perform particular tasks or implement particular abstract data types. Computing system 100 may be practiced in distributed cloud computing environments where tasks are performed by remote processing devices that are linked through a communications network. In a distributed cloud computing environment, program modules may be located in both local and remote computing system storage media including memory storage devices.

As shown in FIG. 1, the computing system 100 has one or more processors 102, which may include, for example, one or more central processing units (CPU), graphics processing units (GPU), or any other processor known in the art. The processors 102 can be a single-core processor, multi-core processor, computing cluster, or any number of other configurations. The processors 102, also referred to as processing circuits, are coupled via a system bus 104 to a system memory 106 and various other components. The system memory 106 can include a read only memory or ROM 108 and a random access memory or RAM 110. The ROM 108 is coupled to the system bus 104 and may include a basic input/output system (BIOS), which controls certain basic functions of the computing system 100. The RAM 110 is read-write memory coupled to the system bus 104 for use by the processors 102. The system memory 106 provides temporary memory space for operations of said instructions during operation. The system memory 106 can include random access memory (RAM), read only memory, flash memory, or any other suitable memory systems.

The computing system 100 comprises an I/O adapter 112 (input/output adapter) and a communications adapter 114 coupled to the system bus 104. The I/O adapter 112 may be a small computer system interface (SCSI) adapter that communicates with a hard disk 116 and/or any other similar component. The I/O adapter 112 and the hard disk 116 are collectively referred to herein as a mass storage 118.

Software 120 for execution on the computing system 100 may be stored in the mass storage 118. The mass storage 118 is an example of a tangible storage medium readable by the processors 102, where the software 120 is stored as instructions for execution by the processors 102 to cause the computing system 100 to operate, such as is described herein below with respect to the various figures. Examples of computer program product and the execution of such instruction is discussed herein in more detail. The communications adapter 114 interconnects the system bus 104 with a network 122, which may be an outside network, enabling the computing system 100 to communicate with other such systems. In one embodiment, a portion of the system memory 106 and the mass storage 118 collectively store an operating system, which may be any appropriate operating system, to coordinate the functions of the various components shown in FIG. 1.

Additional input/output devices are shown as connected to the system bus 104 via a display adapter 124 and an interface adapter 126. In one embodiment, the I/O adapter 112, the communications adapter 114, the display adapter 124 and the interface adapter 126 may be connected to one or more I/O buses that are connected to the system bus 104 via an intermediate bus bridge (not shown). A display 128 (e.g., a screen or a display monitor) is connected to the system bus 104 by the display adapter 124, which may include a graphics controller to improve the performance of graphics intensive applications and a video controller. A keyboard 130, a mouse 132, among other input/output devices, can be interconnected to the system bus 104 via the interface adapter 126, which may include, for example, a Super I/O chip integrating multiple device adapters into a single integrated circuit. Suitable I/O buses for connecting peripheral devices such as hard disk controllers, network adapters, and graphics adapters typically include common protocols, such as the Peripheral Component Interconnect (PCI). Thus, as configured in FIG. 1, the computing system 100 includes processing capability in the form of the processors 102, and, storage capability including the system memory 106 and the mass storage 118, input means such as the keyboard 130 and the mouse 132, and output capability including the display 128.

In some embodiments, the communications adapter 114 can transmit data using any suitable interface or protocol, such as the internet small computing system interface, among others. The network 122 may be a cellular network, a radio network, a wide area network (WAN), a local area network (LAN), or the Internet, among others. An external computing device may connect to the computing system 100 through the network 122. In some examples, an external computing device may be an external webserver or a cloud computing node.

It is to be understood that the block diagram of FIG. 1 is not intended to indicate that the computing system 100 is to include all of the components shown in FIG. 1. Rather, the computing system 100 can include any appropriate fewer or additional components not illustrated in FIG. 1 (e.g., additional memory components, embedded controllers, modules, additional network interfaces, etc.). Further, the embodiments described herein with respect to computing system 100 may be implemented with any appropriate logic, wherein the logic, as referred to herein, can include any suitable hardware (e.g., a processor, an embedded controller, or an application specific integrated circuit, among others), software (e.g., an application, among others), firmware, or any suitable combination of hardware, software, and firmware, in various embodiments.

FIG. 2 illustrates a method 200 for predicting a fatigue response of a material according to an aspect of the present disclosure. The method 200 may be implemented, for example, in conjunction with the computing system 100 illustrated in FIG. 1. Block 202 of the method 200 involves receiving a user input specifying values of one or more surface roughness parameters. Herein, the user may provide input on one or more roughness parameters that are typically used to characterize the surface of the material for which fatigue life is to be predicted. The one or more parameters may be selected, for example, from the non-limiting examples of surface roughness parameters provided below. It is to be appreciated that the disclosed techniques may be used to account for additional or different parameters.

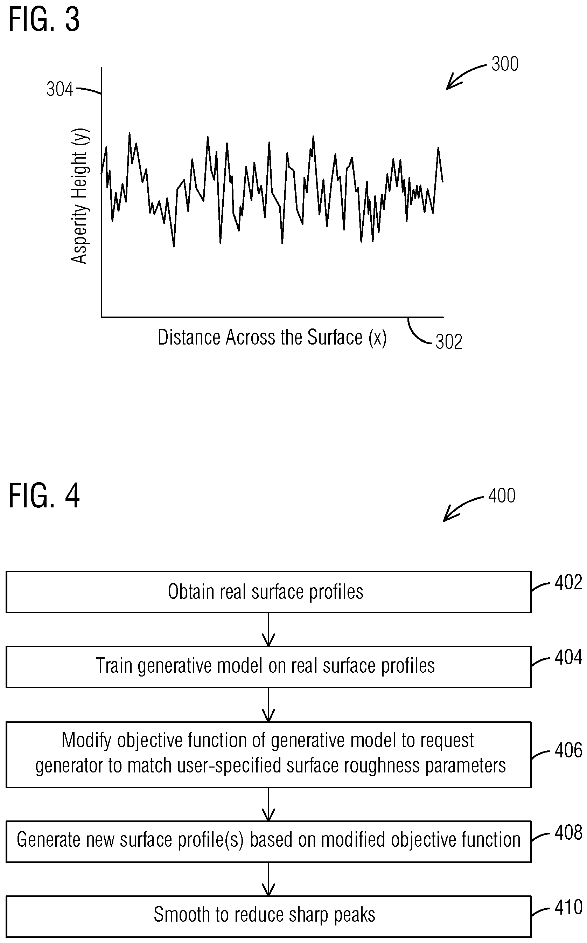

FIG. 3 shows an illustrative one-dimensional (1D) surface curve 300 defining a surface having microscopic asperities, where the axis 302 represents a distance across the surface (x) and the axis y represents asperity height (y).

Assuming the profile is represented as a set of ($x_i$, $y_i$) points, the surface roughness parameters can be defined as:

- Arithmetical mean deviation: $R_a = \frac{1}{n}\sum_{i=1}^{n}|y_i|$ ,, where $y_i$ is the height value at a single point of a 1D surface profile;

- Root mean squared: $R_q = \sqrt{\frac{1}{n}\sum_{i=1}^{n} y_i^2}$

- Maximum valley depth: $R_v = \min_{i} y_i$

- Maximum peak height: $R_p = \max_{i} y_i$

- Maximum height of the profile: $R_t = R_p - R_v$

- Average maximum valley depth: $R_{vm} = \frac{1}{5}\sum_{i=1}^{5}|R_{vi}|$

- Average maximum peak height: $R_{pm} = \frac{1}{5}\sum_{i=1}^{5}|R_{pi}|$

- Skewness: $R_{sk} = \frac{1}{nR_q^3}\sum_{i=1}^{n} y_i^3$

- Kurtosis: $R_{ku} = \frac{1}{nR_q^4}\sum_{i=1}^{n} y_i^4$

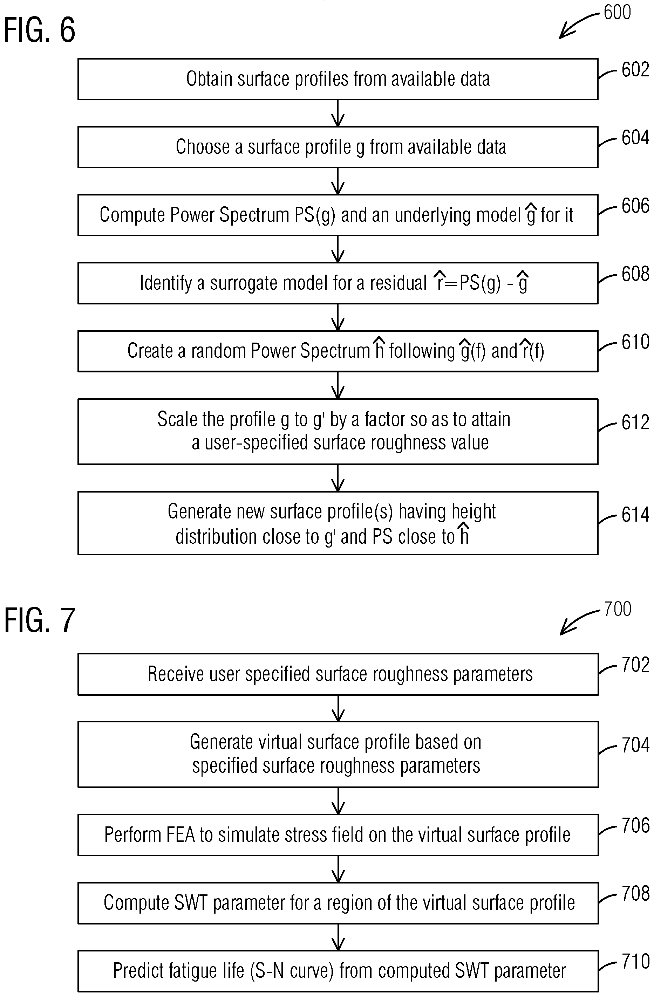

Referring back to FIG. 2, block 204 of the method 200 involves generating at least one realistic virtual surface profile from the specified one or more surface roughness parameters. In the context of the present specification, a "virtual surface profile" refers to a machine generated surface profile obtained from user-specified surface roughness parameter values using a data-driven generative method. In this regard, a virtual surface profile is an artificial surface profile, and is not to be construed as being generated from surface measurements of a real surface. However, a virtual surface may be generated to closely resemble real surface profiles in an available data set. Accordingly, such a virtual surface profile is also described as being "realistic", while not being real. Example embodiments data-driven approaches for generating virtual surface profiles are illustrated below referring to FIG. 4-6. Continuing with reference to FIG. 2, block 206 of the method 200 involves predicting fatigue life of the material based on a stress field applied to the generated virtual surface profile. This may comprise generating a stress-life (S-N curve) plotting cyclic stress amplitude S against fatigue life $N_f$ defined by number of cycles to failure. The generated S-N curve may be graphically displayed on a display screen. Exemplary non-limiting implementations within the scope of the above-described method 200 are illustrated below referring to FIG. 7-9.

FIG. 4 illustrates a first example method 400 for generating one or more virtual surface profiles based on the user-specified surface roughness parameters. The method 400 utilizes a machine learning based generative model. Examples of such models include Generative Adversarial Networks (GAN), Variational Auto-Encoders, or their variations (e.g. WGAN-GP), among others. The generative model may be trained on the available data and utilized to generate novel virtual surface profiles that resemble the surface profiles in the available data. In the shown example, the method 400 utilizes a GAN.

Block 402 of the method 400 involves obtaining, as training data, 1D surface profiles *s* from surface measurements and calculated roughness parameters *r* of a plurality of real surfaces. The data from real surfaces may be pre-processed, which may, for example, involve the steps of resampling the data to obtain same sampling rate throughout all the samples, splitting the data to obtain the same sample length, detrending, etc. Block 404 of the method 400 involves training the generative model using the training data obtained at block 402. At block 406, the generative model is modified to request to the generator to match one or more of the surface roughness parameters specified by the user. In the case of GAN, this can be achieved, for example, by modifying the objective function that the process optimizes by adding the following term:

$$\sum_{i=1}^{9} \mathrm{E}\{[\mathrm{R}_i(\tilde{\mathbf{s}}) - \mathrm{R}_i(\mathbf{s})]^2\}$$

In the above term, $\tilde{\mathbf{s}} = \mathrm{G}(\mathbf{z}, \mathbf{r})$ are the generated 1D surface profiles, as function of the generator G, $\mathbf{z}$ is latent vector and $\mathrm{R}_i(*)$ is the function that represents the roughness parameters $i$. The objective function E is summed over the number of surface roughness parameters specified, which in this case is 9.

Block 408 of the method 400 involves the generation of a specified number of new (virtual) 1D surface profiles $\tilde{\mathbf{s}}$, that closely resemble the surface profiles $\mathbf{s}$ in the training data, based on the modified objective function of the generative model. As an additional step, at block 410, the generated virtual 1D surface profile may be smoothed to reduce sharp peaks, for example, using spectral or spline interpolation.

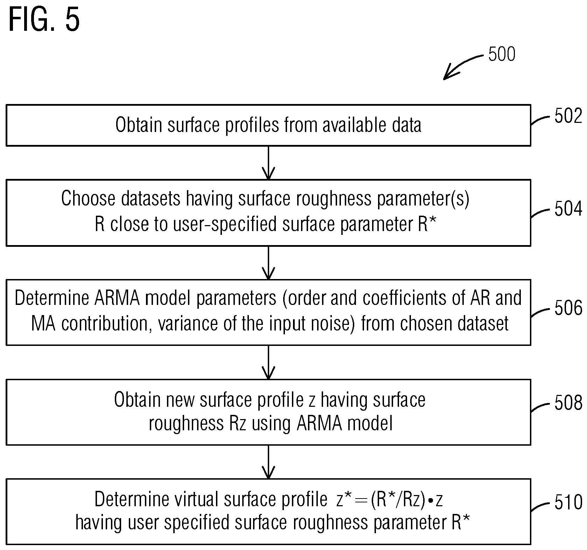

FIG. 5 illustrates a second example method 500 for generating one or more virtual surface profiles based on the user-specified surface roughness parameters. The method 500 uses an Autoregressive Moving Average (ARMA) model to generate new surface profiles. The basic principle of this approach is to identify a suitable mathematical model describing the surface height profile from the available data and using the same model to generate new surface profiles.

Block 502 of the method 500 involves obtaining surface profiles from available data. The available data comprises surface profiles obtained from surface measurements and calculated roughness parameters of a plurality of real surfaces. The data from real surfaces may be pre-processed, which may, for example, involve the steps of resampling the data to obtain same sampling rate throughout all the samples, splitting the data to obtain the same sample length, detrending, etc. Block 504 of the method 500 involves choosing datasets having surface roughness parameters R that are already close to the user-specified surface roughness parameters R*.

Block 506 of the method of the method 500 involves determining a mathematical model of the following form from the chosen dataset.

$$y_t = \sum_{i=1}^{p} a_i y_{t-i} + \sum_{j=1}^{q} b_j \epsilon_{t-j} + \epsilon_t$$

In the above model, $y_t$ denotes the surface height at spatial position $x_t$ and the coefficients $a_i$ and $b_j$ determine the autoregressive (AR) and moving average (MA) part of the model, respectively. Furthermore, the quantity $\varepsilon_t$ denotes white noise, i.e. a random input with mean zero and fixed variance $\sigma^2$, at position $x_t$.

The following model parameters are determined from the chosen dataset, namely order of the AR and MA contribution $p, q$, coefficients of the AR and MA contribution $a_1, ..., a_p, b_1, ..., b_q$ and variation of the input noise $\sigma^2$. For each dataset, these quantities may be identified using, for example, a Box-Jenkins approach in combination with a minimization of Akaike's Information Criterion (AIC). The Box-Jenkins approach is discussed in the publication: Box, George, & Jenkins, Gwilym (1970). Time Series Analysis: Forecasting and Control. San Francisco: Holden-Day. The Akaike's Information Criterion (AIC) is discussed in the publication: Hyndman, R.J., & Athanasopoulos, G. (2018) Forecasting: principles and practice, 2nd edition, OTexts: Melbourne, Australia. OTexts.com/fpp2. Accessed on August 29, 2019.

Under the assumption that there is a single model describing all surface profiles, the aforementioned model parameters may be obtained by averaging the corresponding results from all datasets. The result is an ARMA model describing the spatial evolution of the surface height profile.

Block 508 of the method 500 involves obtaining one or more new surface profiles z using the ARMA model developed in block 506. The new surface profile z may be obtained by specifying an initial height at $x_0 = 0$ (for simplicity this can be assumed to be zero) and creating a series of random white noise input $\varepsilon_t$ with variance $\sigma^2$. As would be expected, the new surface profile z would have surface roughness parameter parameters Rz, where Rz≈R*. Finally, at block 510 of the method 500, a virtual surface z* is generated which would have the user-specified surface roughness parameter R*. The surface profile z* is determined as z* = (R*/Rz) · z.

FIG. 6 illustrates a third example method 600 for generating one or more virtual surface profiles based on the user-specified surface roughness parameters. While the method 500 shown in FIG. 5 works directly on the height data of the surface profiles, the method 600 shown in FIG. 6 identifies typical surface height frequency patterns from available data and creating new (virtual) surface profiles based on those patterns.

Block 602 of the example method 600 involves obtaining surface profiles from available data. The available data comprises surface profiles obtained from surface measurements and calculated roughness parameters of a plurality of real surfaces. The data from real surfaces may be pre-processed, which may, for example, involve the steps of resampling the data to obtain same sampling rate throughout all the samples, splitting the data to obtain the same sample length, detrending, etc. Block 604 of the method 600 involves choosing a surface profile g from the available data. A particular strategy for the choice is presented at block 612. Block 606 of the method 600 involves computing a Power Spectrum (PS) of g, namely, $PS(g)$, and an underlying model $\hat{g}$ for it via the nonlinear least squares (NLS) method. It has been observed that the following parameterized model presents a good candidate.

$$\hat{g}(f) = (af^3 + bf^2 + cf) \cdot \exp(-\beta f)$$

In the above model, the parameters *a, b, c,* $\beta$ may be identified by NLS.

Block 608 of the method 600 involves identifying a surrogate model for the residual or noise term $\hat{r} = PS(g) - \hat{g}$, again via NLS. Typically, $\hat{r}$ is normally distributed with variance decaying exponentially for increasing frequencies. This is due to the fact that such frequencies larger than a certain threshold value are essentially non-existent. This decay motivates a model of the form

$$\hat{r}(f) \sim N(0, \sigma_{\hat{r}}(f)^2)$$

In the above model $\sigma_{\hat{r}}(f) = \gamma \cdot \exp(-\delta \cdot f)$. It can be seen that the variance decreases if the frequency *f* increases.

Block 610 of the method 600 involves creating a random Power Spectrum $\hat{h}$ following the trend $\hat{g}(f)$ and a noise term $\hat{r}(f)$ from above-mentioned distribution. The generated surface profile will eventually have a PS close to $\hat{h}$.

Block 612 of the method 600 involves scaling the profile *g* by a factor $\omega$ such that $g' = \omega \cdot g$ attains a desired surface roughness value as specified by the user. In order to minimize the distortion in the data, in block 604, the surface *g* should be chosen as the one having a roughness value that is closest to the desired surface roughness among all datasets.

Block 614 of the method 600 involves generating one or more new (virtual) surface profiles such that the new surface profile has (a) the same height distribution and hence the same surface roughness as *g'*, and (b) a Power Spectrum that is very close to $\hat{h}$ and is therefore assumed to be realistic as $\hat{h}$ was identified from the data. The new surface profile(s) may be generated using an iterative version of the amplitude adjusted Fourier transform (AAFT). A discussion on AAFT is available in the publication: Schreiber, Thomas, & Andreas Schmitz. "Improved surrogate data for nonlinearity tests." Physical Review Letters 77.4 (1996): 635.

One or more of the methods illustrated in FIG. 4-6 may be used, individually, or in combination, to generate new (virtual) surface profiles. For example, the methods of FIG. 4 and 6 may be combined to achieve machine learning generated models that resemble the training data and also achieve the frequency patterns extracted from the available data. A first way to accomplish the above is to proceed with the method 400 shown in FIG. 4, and then (after block 410) add additional blocks as described for the method 600 of FIG. 6. A second way to accomplish the above is to add a term in the objective function of the generative model (see block 406 of FIG. 4) to penalize in the generative process surface profiles whose frequency content differ from the desired one.

Referring now to FIG. 7, a first example implementation is described of a method 700 for predicting fatigue response of a material based on surface roughness. At block 702 of the method 700, a user input is received specifying values of one or more surface roughness parameters characterizing a surface of the material for which fatigue life is to be predicted. The specified surface roughness parameters may include one or more of the surface roughness parameters described above, or may include additional or different surface roughness parameters. Block 704 of the method 700 involves generating at least one virtual surface profile based on the surface roughness parameters specified in block 702. The virtual surface profile may be generated using one or more of the techniques illustrated above referring to FIG. 4-6.

At block 706, the method 700 involves creating a simulation model using finite element analysis (FEA), based on the generated virtual surface profile. The method includes applying appropriate loads and constraints to the FEA model to simulate a stress field on the virtual surface. In some embodiments, the simulated stress field may be graphically displayed on a display screen. While at a global level, the material may be assumed to exhibit a linear elastic behavior, at a local level, the material may exhibit plastic behavior due to stress concentration effects caused by surface roughness, porosity, etc. Accordingly, at block 708, the method 700 computes a Smith-Watson-Topper (SWT) parameter to account for the localized plastic behavior, in order to predict fatigue life of the material. The SWT parameter, is known in the art (see Smith, K. N., Watson, P. and Topper, T. H. (1970) A stress-strain function for the fatigue of materials. J. Mater. 5, 767-778.), and is defined by the following equation:

$$\frac{\Delta\varepsilon}{2}\sigma_{max} = \frac{(\sigma'_f)^2}{E}(2N_f)^{2b} + \sigma'_f\varepsilon'_f(2N_f)^{b+c}$$

In the above equation, the term on the left is the SWT parameter, which is a product of the maximum stress $\sigma_{max}$ and the strain value $\Delta\varepsilon/2$ for that said maximum stress. In the expression on the right, $N_f$ denotes fatigue life defined by number of cycles to failure, *b* is the Basquin exponent, *c* is the Coffin-Mansion exponent, and $\sigma'_f$, *E* and $\varepsilon'_f$ are constants.

The values $\sigma_{max}$ and $\Delta\varepsilon/2$ may be computed from the FEA model, to thereby determine a value of the SWT parameter for a region of the virtual surface profile, which may be then used to determine fatigue life $N_f$ based on the above equation.

Finally, at block 710 of the method 700, a fatigue life of the material is predicted based on the computed SWT parameter. This may comprise generating an S-N curve plotting cyclic stress amplitude S against fatigue life $N_f$ defined by number of cycles to failure. The generated S-N curve may be graphically displayed on a display screen. The computed SWT parameters may be related to the S-N curve through the well-known Basquin-Coffin-Mansion equation.

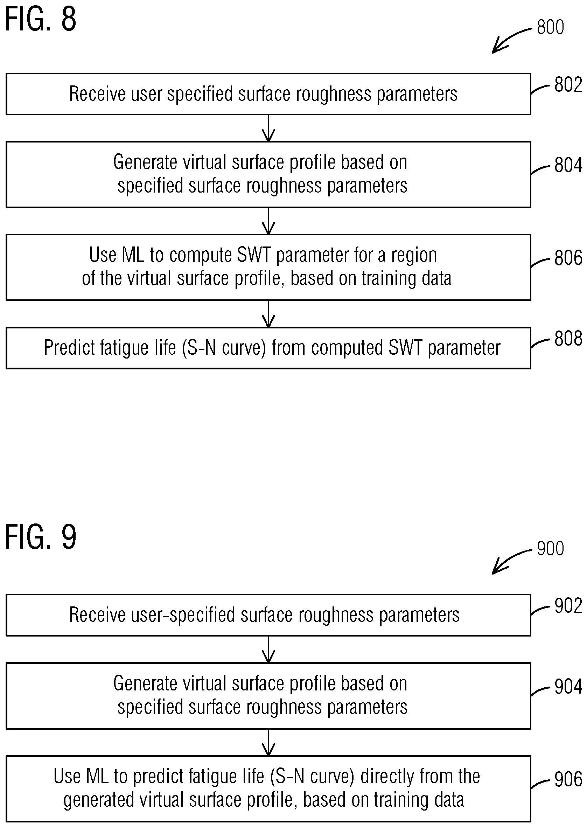

FIG. 8 illustrates a second example implementation of a method 800 for predicting fatigue response of a material based on surface roughness. The proposed method 800 uses a combination of machine learning (ML) and FEA based simulation to speed up the computation of the SWT parameter in order to predict fatigue life.

At block 802 of the method 800, a user input is received specifying values of one or more surface roughness parameters characterizing a surface of the material for which fatigue life is to be predicted. The specified surface roughness parameters may include one or more of the surface roughness parameters described above, or may include additional or different surface roughness parameters. Block 804 of the method 800 involves generating at least one virtual surface profile based on the surface roughness parameters specified in block 802. The virtual surface profile may be generated using one or more of the techniques illustrated above referring to FIG. 4-6.

At block 806 of the method 800, a value of the SWT parameter for a region of the virtual surface profile is determined using an ML based model. The ML based model is trained on previously generated data points pertaining to a large number of sample virtual surface profiles. Each training data point is generated by simulating a stress field on a respective sample virtual surface profile using FEA, and computing therefrom an SWT parameter for a region of the sample virtual surface profile, similar to that described in connection with blocks 706 and 708 of FIG. 7. The ML based model of FIG. 8 may be thus trained to map the input surface profile to the result of the finite element simulation (i.e., the computed value of the SWT parameter). To this end, the method 700 of FIG. 7 may be utilized for generating the training data points for implementing the method 800 of FIG. 8. Once a sufficiently large number of data points are generated using the method 700, the method 800 may be implemented, to accelerate the computation of the SWT parameter.

At block 808 of the method 800, a fatigue life of the material is predicted based on the computed SWT parameter. This may comprise generating an S-N curve plotting cyclic stress amplitude S against fatigue life $N_f$ defined by number of cycles to failure. The generated S-N curve may be graphically displayed on a display screen. The computed SWT parameters may be related to the S-N curve through the well-known Basquin-Coffin-Mansion equation. In case of the method 800, the determination of the predicted fatigue life is greatly simplified by computing the SWT parameter directly based on ML, bypassing the more time-consuming FEA based approach.

FIG. 9 illustrates a third example implementation of a method 900 for predicting fatigue response of a material based on surface roughness. The proposed method 900 utilizes ML to predict fatigue life directly from the generated virtual surface profile.

At block 902 of the method 900, a user input is received specifying values of one or more surface roughness parameters characterizing a surface of the material for which fatigue life is to be predicted. The specified surface roughness parameters may include one or more of the surface roughness parameters described above, or may include additional or different surface roughness parameters. Block 904 of the method 900 involves generating at least one virtual surface profile based on the surface roughness parameters specified in block 902. The virtual surface profile may be generated using one or more of the techniques illustrated above referring to FIG. 4-6.

At block 906 of the method 900, the fatigue life of the material is predicted (and may also be graphically displayed on a display screen), for example, in the form of an S-N curve, directly from the generated virtual surface profile, using the ML based model. The ML based model is trained on previously generated data points pertaining to a large number of sample virtual surface profiles. Each training data point is generated by simulating a stress field on a respective sample virtual surface profile using FEA, and computing therefrom an SWT parameter for a region of the sample virtual surface profile, and determining fatigue life (S-N curve) corresponding to the sample virtual surface profile from the computed SWT parameter. The ML based model of FIG. 9 may be thus trained to map the input surface profile to the predicted S-N curve.

To generate the training data points for implementing the method 900, the method 700 (FIG. 7) and the method 800 (FIG. 8) may be utilized. The implementation of the method 900 is based on the understanding that the simulated stress fields contain enough information to be able to predict when a component would fail. However, this might involve a large amount of data for the ML based model to process and learn patterns from. Accordingly, the method 900 may be implemented only after a sufficiently large number of data points are generated linking the stress fields to the eventual S-N curves. The methods 700 and 800 may be utilized to allow the ML based model to reach to the required level of learning.

Aspects of the present disclosure may include a system, a method, and/or a computer program product at any possible technical detail level of integration. The computer program product may include a computer readable storage medium (or media) having computer readable program instructions thereon for causing a processor to carry out aspects of the present disclosure. A computer readable storage medium, as used herein, is understood to be a non-transitory storage medium, which is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Aspects of the present disclosure are described herein with reference to flowchart illustrations and/or block diagrams of methods, apparatus (systems), and computer program products according to embodiments of the disclosure. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The functions and process steps herein may be performed automatically, wholly or partially in response to user command. An activity (including a step) performed automatically is performed in response to one or more executable instructions or device operation without user direct initiation of the activity.

The system and processes of the figures are not exclusive. Other systems and processes may be derived in accordance with the principles of the disclosure to accomplish the same objectives. Although this disclosure has been described with reference to particular embodiments, it is to be understood that the embodiments and variations shown and described herein are for illustration purposes only. Modifications to the current design may be implemented by those skilled in the art, without departing from the scope of the disclosure. The invention is defined by the appended independent claims. The dependent claims define preferred embodiments.

**Claims**

1. A computer-implemented method (200) for predicting a fatigue response of a material, comprising:

   Receiving a user input specifying one or more surface roughness parameters that characterize a surface of a material for which fatigue life is to be predicted (202),
   generating at least one realistic artificial virtual surface profile from the specified one or more surface roughness parameters using a data-driven generative method to closely resemble real surface profiles (204) by a machine learning based generative model (400), the machine learning based generative model being developed using training data (404) comprising surface profiles obtained from surface measurements and calculated roughness parameters of a plurality of real surfaces (402), and
   predicting fatigue life of the material in dependence of a stress field applied to the generated virtual surface profile (206).

**2.** The method according to claim 1, wherein the at least one virtual surface profile is generated by an Autoregressive Moving Average, "ARMA", model (500), the ARMA model being developed to describe a surface height profile (510) from available data comprising surface profiles obtained from surface measurements and calculated roughness parameters of a plurality of real surfaces (502).

**3.** The method according to any of claims 1 and 2, wherein the at least one virtual surface profile is generated based on surface height frequency patterns (600) identified from available data comprising surface profiles obtained from surface measurements and calculated roughness parameters of a plurality of real surfaces (602).

**4.** The method according to any of claims 1 to 3, wherein the prediction of the fatigue life of the material is based on a computation of a Smith-Watson-Topper, "SWT", parameter from the applied stress field on the virtual surface profile (700).

**5.** The method according to claim 4, wherein the computation of the SWT parameter (700) comprises performing a finite element analysis to generate a simulated stress field on the virtual surface profile (706), and determining a value of the SWT parameter for a region of the virtual surface profile (708).

**6.** The method according to claim 4, wherein the computation of the SWT parameter comprises determining a value of the SWT parameter for a region of the virtual surface profile using a machine learning based model (800), Wherein the machine learning based model is trained on data points pertaining to a plurality of sample virtual surface profiles, each training data point being generated by performing finite element analysis to simulate a stress field on a respective sample virtual surface profile, and compute therefrom an SWT parameter for a region of the sample virtual surface profile (806).

**7.** The method according to any of claims 4 to 6, wherein predicting the fatigue life of the material comprises generating a stress-life (S-N) curve from the computed SWT parameter (710, 808).

**8.** The method according to any of claims 1 to 3, wherein the fatigue life of the material is predicted based on a machine learning based model trained to predict fatigue life directly from the generated virtual surface profile (900), wherein the machine learning based model is trained on data points pertaining to a plurality of sample virtual surface profiles, each training data point being generated by:

performing finite element analysis to simulate a stress field on a respective sample virtual surface profile, and compute therefrom a Smith-Watson-Topper (SWT) parameter for a region of the sample virtual surface profile, and
determining fatigue life corresponding to the sample virtual surface profile from the computed SWT parameter.

**9.** The method according to claim 8, predicting the fatigue life of the material comprises generating a stress-life (S-N) curve using the machine learning based model (906).

**10.** A non-transitory computer-readable storage medium (118) including instructions that, when processed by a computer, configure the computer to perform the method (200) according to any of claims 1 to 9.

**11.** A computing system (100) comprising:

a processor (102); and
a memory storing (106) instructions that, when executed by the processor (102), configure the computing system (100) to:

receive a user input specifying one or more surface roughness parameters that characterize a surface of a material for which fatigue life is to be predicted (202),
generate at least one realistic artificial virtual surface profile from the specified one or more surface roughness parameters using a data-driven generative method to closely resemble real surface profiles (204) by a machine learning based generative model (400), the machine learning based generative model being developed using training data (404) comprising surface profiles obtained from surface measurements and calculated roughness parameters of a plurality of real surfaces (402), and
predict fatigue life of the material in dependence of a stress field applied to the generated virtual surface profile (206).

12. The computing system according to claim 11, wherein the at least one realistic virtual surface profile is generated by a machine learning based generative model, the machine learning based generative model being developed using training data comprising surface profiles obtained from surface measurements and calculated roughness parameters of a plurality of real surfaces (400).

13. The computing system according to any of claims 11 and 12, wherein the at least one virtual surface profile is generated by an Autoregressive Moving Average, "ARMA", model, the ARMA model being developed to describe a surface height profile from available data comprising surface profiles obtained from surface measurements and calculated roughness parameters of a plurality of real surfaces (500).

14. The computing system according to any of claims 11 to 13, wherein the at least one virtual surface profile is generated based on surface height frequency patterns identified from available data comprising surface profiles obtained from surface measurements and calculated roughness parameters of a plurality of real surfaces (600).

**Patentansprüche**

1. Computerimplementiertes Verfahren (200) zum Vorhersagen einer Ermüdungsreaktion eines Materials, umfassend:

   Empfangen einer Benutzereingabe, die einen oder mehrere Oberflächenrauheitparameter spezifiziert, die eine Oberfläche eines Materials kennzeichnen, dessen Ermüdungslebensdauer vorhergesagt werden soll (202), Erzeugen mindestens eines realistischen künstlichen virtuellen Oberflächenprofils aus dem spezifizierten einen oder den spezifizierten mehreren Oberflächenrauheitparametern unter Einsatz eines Datengesteuerten generativen Verfahrens zum genauen Ähneln von realen Oberflächenprofilen (204) über ein auf Maschinenlernen basierendes generatives Modell (400), wobei das auf Maschinenlernen basierende generative Modell entwickelt wird unter Einsatz von Trainingsdaten (404), umfassend aus Oberflächenmessungen erhaltene Oberflächenprofile sowie berechnete Rauheitsparameter einer Vielzahl von realen Oberflächen (402), und Vorhersagen der Ermüdungslebensdauer des Materials in Abhängigkeit eines Belastungsfelds, das auf das erzeugte virtuelle Oberflächenprofil angelegt wird (206).

2. Verfahren nach Anspruch 1, wobei das mindestens eine virtuelle Oberflächenprofil erzeugt wird durch ein ARMA-Modell (Autoregressive Moving Average) (500), wobei das ARMA-Modell entwickelt worden ist, um ein Oberflächenhöhenprofil (510) aus verfügbaren Daten zu beschreiben, umfassend aus Oberflächenmessungen erhaltene Oberflächenprofile sowie berechnete Rauheitsparameter einer Vielzahl von realen Oberflächen (502).

3. Verfahren nach einem der Ansprüche 1 und 2, wobei das mindestens eine virtuelle Oberflächenprofil erzeugt wird auf der Grundlage von Oberflächenhöhenfrequenzmustern (600), identifiziert aus verfügbaren Daten, umfassend aus Oberflächenmessungen erhaltene Oberflächenprofile sowie berechnete Rauheitsparameter einer Vielzahl von realen Oberflächen (602).

4. Verfahren nach einem der Ansprüche 1 bis 3, wobei das Vorhersagen der Ermüdungslebensdauer des Materials auf einer Berechnung eines SWT(Smith-Watson-Topper)-Parameters von dem angelegten Belastungsfeld auf dem virtuellen Oberflächenprofil basiert (700).

5. Verfahren nach Anspruch 4, wobei das Berechnen des SWT-Parameters (700) das Ausführen einer finiten Elementanalyse, um ein simuliertes Belastungsfeld auf dem virtuellen Oberflächenprofil (706) zu erzeugen, sowie das Bestimmen eines Wertes des SWT-Parameters für eine Region des virtuellen Oberflächenprofils (708) umfasst.

6. Verfahren nach Anspruch 4, wobei das Berechnen des SWT-Parameters das Bestimmen eines Werts des SWT-Parameters für eine Region des virtuellen Oberflächenprofils unter Einsatz eines auf Maschinenlernen basierenden Modells umfasst (800), wobei das auf Maschinenlernen basierende Profil auf Datenpunkten trainiert ist, die sich auf eine Vielzahl von virtuellen Probenoberflächenprofilen beziehen, wobei jeder Trainingsdatenpunkt erzeugt wird durch Ausführen einer finiten Elementanalyse zum Simulieren eines Belastungsfelds auf einem virtuellen Probenoberflächenprofil und daraus Berechnen eines SWT-Parameters für eine Region des virtuellen Probenoberflächenprofils (806).

7. Verfahren nach einem der Ansprüche 4 bis 6, wobei das Vorhersagen der Ermüdungslebensdauer des Materials das Erzeugen einer Kurve der Ermüdungslebensdauer (S-N) aus dem berechneten SWT-Parameter umfasst (710, 808).

8. Verfahren nach einem der Ansprüche 1 bis 3, wobei die Ermüdungslebensdauer des Materials auf Grundlage eines auf Maschinenlernen basierenden Modells vorhergesagt wird, das dazu trainiert worden ist, Ermüdungslebensdauer direkt aus dem erzeugten virtuellen Oberflächenprofil vorherzusagen (900), wobei das auf Maschinenlernen basierende Profil auf Datenpunkten trainiert ist, die sich auf eine Vielzahl von virtuellen Probenoberflächenprofilen beziehen, wobei jeder Trainingsdatenpunkt erzeugt wird durch:

Ausführen einer finiten Elementanalyse zum Simulieren eines Belastungsfelds auf einem virtuellen Probenoberflächenprofil und daraus Berechnen eines SWT(Smith-Watson-Topper)-Parameters für eine Region des virtuellen Probenoberflächenprofils, und

Bestimmen der Ermüdungslebensdauer, die dem virtuellen Probenoberflächenprofil entspricht, aus dem berechneten SWT-Parameter.

9. Verfahren nach Anspruch 8, das Vorhersagen der Ermüdungslebensdauer des Materials umfasst das Erzeugen einer Kurve der Ermüdungslebensdauer (S-N) unter Einsatz des auf Maschinenlernen basierenden Profils (906).

10. Nicht-flüchtiges computerlesbares Speichermedium (118), das Anweisungen beinhaltete, die, wenn sie durch einen Computer verarbeitet werden, den Computer zum Durchführen des Verfahrens (200) nach einem der Ansprüche 1 bis 9 konfigurieren.

11. Rechensystem (100), umfassend:

einen Prozessor (102); und
einen Speicher (106), in dem Anweisungen gespeichert sind, die, wenn durch den Prozessor (102) ausgeführt, das Rechensystem (100) konfigurieren zum:

Empfangen einer Benutzereingabe, die einen oder mehrere Oberflächenrauheitparameter spezifiziert, die eine Oberfläche eines Materials kennzeichnen, dessen Ermüdungslebensdauer vorhergesagt werden soll (202),
Erzeugen mindestens eines realistischen künstlichen virtuellen Oberflächenprofils aus dem spezifizierten einen oder den spezifizierten mehreren Oberflächenrauheitparametern unter Einsatz eines Datengesteuerten generativen Verfahrens zum genauen Ähneln von realen Oberflächenprofilen (204) über ein auf Maschinenlernen basierendes generatives Modell (400), wobei das auf Maschinenlernen basierende generative Modell entwickelt wird unter Einsatz von Trainingsdaten (404), umfassend aus Oberflächenmessungen erhaltene Oberflächenprofile sowie berechnete Rauheitsparameter einer Vielzahl von realen Oberflächen (402), und
Vorhersagen der Ermüdungslebensdauer des Materials in Abhängigkeit eines Belastungsfelds, das auf das erzeugte virtuelle Oberflächenprofil angelegt wird (206).

12. Rechensystem nach Anspruch 11, wobei das mindestens eine realistische virtuelle Oberflächenprofil erzeugt wird durch ein aus Maschinenlernen basierendes generatives Modell, wobei das auf Maschinenlernen basierende generative Modell entwickelt wird unter Einsatz von Trainingsdaten, umfassend aus Oberflächenmessungen erhaltene Oberflächenprofile sowie berechnete Rauheitsparameter einer Vielzahl von realen Oberflächen (400).

13. Rechensystem nach einem der Ansprüche 11 und 12, wobei das mindestens eine virtuelle Oberflächenprofil erzeugt wird durch ein ARMA-Modell (Autoregressive Moving Average), wobei das ARMA-Modell entwickelt worden ist, um ein Oberflächenhöhenprofil aus verfügbaren Daten zu beschreiben, umfassend aus Oberflächenmessungen erhaltene Oberflächenprofile sowie berechnete Rauheitsparameter einer Vielzahl von realen Oberflächen (500).

14. Rechensystem nach einem der Ansprüche 11 bis 13, wobei das mindestens eine virtuelle Oberflächenprofil erzeugt wird auf der Grundlage von Oberflächenhöhenfrequenzmustern, identifiziert aus verfügbaren Daten, umfassend aus Oberflächenmessungen erhaltene Oberflächenprofile sowie berechnete Rauheitsparameter einer Vielzahl von realen Oberflächen (600).

**Revendications**

1. Procédé mis en œuvre par ordinateur (200) pour prédire une réponse à la fatigue d'un matériau, comprenant :

la réception d'une entrée utilisateur spécifiant un ou plusieurs paramètres de rugosité de surface qui caractérisent une surface d'un matériau pour lequel la durée de vie en fatigue doit être prédite (202),

la génération d'au moins un profil de surface virtuelle artificielle réaliste à partir du ou des paramètres de rugosité de surface spécifiés à l'aide d'un procédé génératif axé sur les données pour ressembler étroitement à des profils de surface réels (204) par un modèle génératif basé sur l'apprentissage automatique (400), le modèle génératif basé sur l'apprentissage automatique étant développé à l'aide de données d'entraînement (404) comprenant des profils de surface obtenus à partir de mesures de surface et de paramètres de rugosité calculés d'une pluralité de surfaces réelles (402) et

la prédiction de la durée de vie en fatigue du matériau en fonction d'un champ de contrainte appliqué au profil de surface virtuelle généré (206).

2. Procédé selon la revendication 1, l'au moins un profil de surface virtuel étant généré par un modèle de moyenne mobile autorégressive, "ARMA", (500), le modèle ARMA étant développé pour décrire un profil de hauteur de surface (510) à partir de données disponibles comprenant des profils de surface obtenus à partir de mesures de surface et de paramètres de rugosité calculés d'une pluralité de surfaces réelles (502).

3. Procédé selon l'une quelconque des revendications 1 et 2, l'au moins un profil de surface virtuelle étant généré sur la base de modèles de fréquence de hauteur de surface (600) identifiés à partir de données disponibles comprenant des profils de surface obtenus à partir de mesures de surface et de paramètres de rugosité calculés d'une pluralité de surfaces réelles (602).

4. Procédé selon l'une quelconque des revendications 1 à 3, la prédiction de la durée de vie en fatigue du matériau étant basée sur un calcul d'un paramètre de Smith-Watson-Topper, "SWT", à partir du champ de contrainte appliqué sur le profil de surface virtuelle (700).

5. Procédé selon la revendication 4, le calcul du paramètre SWT (700) comprenant la réalisation d'une analyse par éléments finis pour générer un champ de contrainte simulé sur le profil de surface virtuel (706), et la détermination d'une valeur du paramètre SWT pour une région du profil de surface virtuel (708).

6. Procédé selon la revendication 4, le calcul du paramètre SWT comprenant la détermination d'une valeur du paramètre SWT pour une région du profil de surface virtuelle à l'aide d'un modèle basé sur l'apprentissage automatique (800), le modèle basé sur l'apprentissage automatique étant entraîné sur des points de données relatifs à une pluralité d'échantillons de profils de surface virtuelle, chaque point de données d'entraînement étant généré en effectuant une analyse par éléments finis pour simuler un champ de contrainte sur un échantillon respectif de profil de surface virtuelle, et calculer à partir de cela un paramètre SWT pour une région de l'échantillon de profil de surface virtuelle (806).

7. Procédé selon l'une quelconque des revendications 4 à 6, la prédiction de la durée de vie en fatigue du matériau comprenant la génération d'une courbe contrainte-durée de vie (S-N) à partir du paramètre SWT calculé (710, 808).

8. Procédé selon l'une quelconque des revendications 1 à 3, la durée de vie en fatigue du matériau étant prédite sur la base d'un modèle basé sur l'apprentissage automatique entraîné à prédire la durée de vie en fatigue directement à partir du profil de surface virtuelle généré (900),

le modèle basé sur l'apprentissage automatique étant entraîné sur des points de données relatifs à une pluralité d'échantillons de profils de surface virtuelle, chaque point de données d'entraînement étant généré par :

la réalisation d'une analyse par éléments finis pour simuler un champ de contrainte sur un échantillon respectif de profil de surface virtuelle, et en calculant à partir de cela un paramètre Smith-Watson-Topper (SWT) pour une région de l'échantillon de profil de surface virtuelle, et

la détermination de la durée de vie en fatigue correspondant au profil de surface virtuelle de l'échantillon à partir du paramètre SWT calculé.

9. Procédé selon la revendication 8, la prédiction de la durée de vie en fatigue du matériau comprenant la génération d'une courbe contrainte-durée de vie (S-N) à l'aide du modèle (906) basé sur l'apprentissage automatique.

10. Support de stockage non transitoire lisible par ordinateur (118) comprenant des instructions qui, lorsqu'elles sont traitées par un ordinateur, configurent l'ordinateur pour qu'il réalise le procédé (200) selon l'une quelconque des revendications 1 à 9.

**11.** Système informatique (100) comprenant :

un processeur (102) ; et
une mémoire stockant (106) des instructions qui, lorsqu'elles sont exécutées par le processeur (102), configurent le système informatique (100) pour :

recevoir une entrée utilisateur spécifiant un ou plusieurs paramètres de rugosité de surface qui caractérisent une surface d'un matériau pour lequel la durée de vie en fatigue doit être prédite (202),
générer au moins un profil de surface virtuelle artificielle réaliste à partir du ou des paramètres de rugosité de surface spécifiés à l'aide d'un procédé génératif axé sur les données pour ressembler étroitement à des profils de surface réels (204) par un modèle génératif basé sur l'apprentissage automatique (400), le modèle génératif basé sur l'apprentissage automatique étant développé à l'aide de données d'entraînement (404) comprenant des profils de surface obtenus à partir de mesures de surface et de paramètres de rugosité calculés d'une pluralité de surfaces réelles (402), et
prédire la durée de vie en fatigue du matériau en fonction d'un champ de contrainte appliqué au profil de surface virtuelle généré (206).

**12.** Système informatique selon la revendication 11, l'au moins un profil de surface virtuelle réaliste étant généré par un modèle génératif basé sur l'apprentissage automatique, le modèle génératif basé sur l'apprentissage automatique étant développé à l'aide de données d'entraînement comprenant des profils de surface obtenus à partir de mesures de surface et de paramètres de rugosité calculés d'une pluralité de surfaces réelles (400).

**13.** Système informatique selon l'une quelconque des revendications 11 et 12, l'au moins un profil de surface virtuelle étant généré par un modèle de moyenne mobile autorégressive, "ARMA", le modèle ARMA étant développé pour décrire un profil de hauteur de surface à partir de données disponibles comprenant des profils de surface obtenus à partir de mesures de surface et de paramètres de rugosité calculés d'une pluralité de surfaces réelles (500).

**14.** Système informatique selon l'une quelconque des revendications 11 à 13, l'au moins un profil de surface virtuelle étant généré sur la base de modèles de fréquence de hauteur de surface identifiés à partir de données disponibles comprenant des profils de surface obtenus à partir de mesures de surface et de paramètres de rugosité calculés d'une pluralité de surfaces réelles (600).

# FIG. 1

Mass Storage ——118

Hard Disk ——116

Software ——120

I/O Adapter ——112

Processor ——102

Processor ——102

Processor

102

System Memory ——106

ROM    RAM

108 ———110

Communications Adapter ——114

Network ——122

System Bus
104

Interface Adapter ——126

Display Adapter ——124

Keyboard    Mouse

130    132

Display ——128

# FIG. 2

200

Receive user specified surface roughness parameters ——202

Generate virtual surface profile based on specified surface roughness parameters ——204

Predict fatigue life based on stress field applied to the virtual surface profile ——206

# FIG. 3

# FIG. 4

| | |
|---|---|
| Obtain real surface profiles | 402 |
| Train generative model on real surface profiles | 404 |
| Modify objective function of generative model to request generator to match user-specified surface roughness parameters | 406 |
| Generate new surface profile(s) based on modified objective function | 408 |
| Smooth to reduce sharp peaks | 410 |

# FIG. 5

500

| Obtain surface profiles from available data |
|---|

502

| Choose datasets having surface roughness parameter(s) R close to user-specified surface parameter R* |
|---|

504

| Determine ARMA model parameters (order and coefficients of AR and MA contribution, variance of the input noise) from chosen dataset |
|---|

506

| Obtain new surface profile z having surface roughness Rz using ARMA model |
|---|

508

| Determine virtual surface profile $z^* = (R^*/Rz) \cdot z$ having user specified surface roughness parameter R* |
|---|

510

# FIG. 6

600

| Obtain surface profiles from available data | 602 |

↓

| Choose a surface profile g from available data | 604 |

↓

| Compute Power Spectrum PS(g) and an underlying model $\hat{g}$ for it | 606 |

↓

| Identify a surrogate model for a residual $\hat{r}$=PS(g) - $\hat{g}$ | 608 |

↓

| Create a random Power Spectrum $\hat{h}$ following $\hat{g}(f)$ and $\hat{r}(f)$ | 610 |

↓

| Scale the profile g to g' by a factor so as to attain a user-specified surface roughness value | 612 |

↓

| Generate new surface profile(s) having height distribution close to g' and PS close to $\hat{h}$ | 614 |

# FIG. 7

700

| Receive user specified surface roughness parameters | 702 |

↓

| Generate virtual surface profile based on specified surface roughness parameters | 704 |

↓

| Perform FEA to simulate stress field on the virtual surface profile | 706 |

↓

| Compute SWT parameter for a region of the virtual surface profile | 708 |

↓

| Predict fatigue life (S-N curve) from computed SWT parameter | 710 |

# FIG. 8

800

| Receive user specified surface roughness parameters | 802 |

↓

| Generate virtual surface profile based on specified surface roughness parameters | 804 |

↓

| Use ML to compute SWT parameter for a region of the virtual surface profile, based on training data | 806 |

↓

| Predict fatigue life (S-N curve) from computed SWT parameter | 808 |

# FIG. 9

900

| Receive user-specified surface roughness parameters | 902 |

↓

| Generate virtual surface profile based on specified surface roughness parameters | 904 |

↓

| Use ML to predict fatigue life (S-N curve) directly from the generated virtual surface profile, based on training data | 906 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018113967 A1, AGRAWAL ANKIT

**Non-patent literature cited in the description**

- A hybrid approach to the development of a multilayer neural network for wear and fatigue prediction in metal forming. **BELFIORE et al.** TRIBOLOGY INTERNATIONAL. ELSEVIER LTD, 10 August 2007, vol. 40, 1705-1717
- **BOX** ; **GEORGE** ; **JENKINS** ; **GWILYM**. Time Series Analysis: Forecasting and Control. *San Francisco: Holden-Day*, 1970
- **HYNDMAN, R.J.** ; **ATHANASOPOULOS, G.** Forecasting: principles and practice. OTexts, 2018
- **SCHREIBER** ; **THOMAS** ; **ANDREAS SCHMITZ**. Improved surrogate data for nonlinearity tests. *Physical Review Letters*, 1996, vol. 77 (4), 635
- **SMITH, K. N.** ; **WATSON, P.** ; **TOPPER, T. H.** A stress-strain function for the fatigue of materials. *J. Mater.*, 1970, vol. 5, 767-778